# EUROPEAN PATENT APPLICATION

(11) **EP 3 782 650 A1**
(43) Date of publication of application: **24.02.2021**
(21) Application number: 19788597.3
(22) Date of filing: 02.04.2019
(51) Int. Cl.: A61K 47/26, A61K 9/16, A61K 47/36, A61K 47/38, A61K 31/167

(54) **EASY-TO-TAKE GRANULAR PREPARATION, AND METHOD FOR PRODUCING SAME**

(30) Priority: 17.04.2018 JP 2018079472
(71) Applicant: Daicel Corporation, Osaka 530-0011 (JP)
(72) Inventor: MIZUGUCHI tomotaka, Himeji-shi, Hyogo 671-1281 (JP); OKABAYASHI Tomohito, Tokyo 108-8230 (JP); SAKAGUCHI Anan, Himeji-shi, Hyogo 671-1281 (JP); UEDA Momoko, Himeji-shi, Hyogo 671-1281 (JP)
(74) Representative: J A Kemp LLP
(86) International application number: PCT/JP2019/014580
(87) International publication number: WO 2019/202968

(57) **Abstract**

The problem to be solved by the present invention is to provide an easy-to-take granular preparation that forms a gel having low adhesiveness and appropriate hardness when brought into contact with water, exhibiting an improved swallowing as a result, and to provide a method for producing the granular preparation. The present invention pertains to an easy-to-take granular preparation including granules, each of which contains a sugar or a sugar alcohol and has a surface at least partially coated with a thickener, and to a method for producing the easy-to-take granular preparation, the method including granulating a composition that includes a sugar or a sugar alcohol using an aqueous solution of a thickener.

## Description

### Technical Field

The present invention relates to an easy-to-take granular preparation containing granules, characterized in that a surface of each granule is at least partially coated with a substance that turns into a viscous liquid (hereinafter, also simply referred to as a "thickener") when water or the like is added, and thereby the granular preparation forms a gel-like substance having low adhesiveness and an appropriate level of hardness when brought into contact with water. The present invention also relates to a method for producing the easy-to-take granular preparation.

### Background Art

In the past, attempts have been made to improve the swallowability of orally administered formulations for patients who have difficulty swallowing, and children and elderly people with weak swallowing ability. For example, the preparations are formulated into liquid or jelly preparation form in many cases. However, when a content of a main drug is high, it will be difficult to mask its taste, and when an active ingredient such as a drug is unstable in water, it will be difficult to be formulated into any preparation form.

Accordingly, easy-to-take preparations have been recently developed as a means for facilitating swallowing of the solid preparation, where the surface of the preparations is coated with a gelling agent so that they will show slipperiness and easy to swallow when they are brought into contact with water in oral cavity.

For example, Patent Document 1 describes a particle composition that is useful for a solid formulation and contains a sugar alcohol and a water-insoluble polymer, a composition for an easy-to-take solid formulation that contains the particle composition and a gelling agent that becomes slippery when brought into contact with water, and an easy-to-take solid preparation containing the solid formulation composition.

Furthermore, Patent Document 2 describes a particle composition for an easy-to-take solid formulation, the particle composition containing a sugar alcohol and a gelling agent that becomes slippery when brought into contact with water, where a portion or an entirety of its surface of the particle composition is covered with the gelling agent, and also described is an easy-to-take solid formulation containing such a composition.

The easy-to-take preparations containing the particle compositions described in the above-mentioned Patent Document 1 and Patent Document 2 are solid formulations such as tablets that are produced by compression molding.

On the other hand, Patent Document 3 describes granules with improved swallowability, that is, granules having a reduced feeling of roughness in the oral cavity when took, and pills obtained by compression molding the granules using a tableting machine. The granules contain ingredients (such as active ingredients and/or excipients) that are poorly soluble against water or saliva, and ingredients (a disintegrating agent and/or binder such as hydroxypropyl methylcellulose, hydroxypropyl cellulose, and hydroxypropyl starch) that turn into a viscous liquid upon the addition of water. In particular, according to the descriptions of the examples, the granules are produced by mixing these ingredients, adding water to the mixture and kneading, and then granulating the kneaded product using an extrusion granulator and drying the obtained granules.

### Citation List

### Patent Document

Patent Document 1: WO 2017/002803
Patent Document 2: WO 2017/057147
Patent Document 3: WO 2003/026619

### Summary of Invention

### Technical Problem

The granules described in Patent Document 3 have a low degree of dispersion because the ingredient that turns into a viscous liquid is used as a powder to mix with the other ingredients, and granulated after the mixing. As a result, the surface of the aforementioned ingredient with poor solubility is not sufficiently covered with the ingredient that turns into the viscous liquid. Therefore, when the amount of the ingredient that turns into the viscous liquid is large, the powder easily adheres inside the oral cavity when the formulation is put into the mouth. On the other hand, if the amount of such an ingredient is small, its rough texture cannot be sufficiently reduced. As a result, it was not possible to achieve a function of both suppression of rough texture and low adhesiveness.

Thus, an object of the present invention is to solve these problems in the arts, and provide an easy-to-take granular preparation that contains granules containing a sugar or a sugar alcohol with its surface of the granules being at least partially coated by a substance (thickener) that turns into a viscous liquid when water or the like is added, and thereby the granular preparation forms a gel-like substance having low adhesiveness and an appropriate level of hardness when brought into contact with water. Another object of the present invention is to provide a method for producing the granules, Note that in the present invention, "easy-to-take" generally means that as a property and characteristic of an oral agent, the oral agent is easy to drink (easy to swallow).

None of the above-mentioned patent documents specifically describe or suggest such a technical problem.

### Solution to Problem

The present inventors have earnestly studied to solve the above problems and completed the invention comprising the following aspects.

Thus, the present invention provides the following aspects.

### [First Aspect]

An easy-to-take granular preparation including a granule that contains a sugar or a sugar alcohol, the granule having a surface at least partially coated with a thickener.

### [Second Aspect]

The easy-to-take granular preparation according to the first aspect, further including an active ingredient.

### [Third Aspect]

The easy-to-take granular preparation according to the second aspect, including an active ingredient as a constituent element separate from the granule.

### [Fourth Aspect]

The easy-to-take granular preparation according to the second or third aspect, further including an active ingredient in a granule coated with a thickener.

### [Fifth Aspect]

The easy-to-take granular preparation according to any one of the first to fourth aspects, wherein substantially the entire surface of the granule is coated with a thickener.

### [Sixth Aspect]

The easy-to-take granular preparation according to any one of the first to fifth aspects, wherein the thickener includes at least one water-soluble polymer selected from the group consisting of sodium carboxymethyl cellulose, xanthan gum, sodium alginate, carrageenan, guar gum, and gelatin.

### [Seventh Aspect]

The easy-to-take granular preparation according to the sixth aspect, wherein the water-soluble polymer is sodium carboxymethyl cellulose.

### [Eighth Aspect]

The easy-to-take granular preparation according to any one of the first to seventh aspects, wherein the sugar or sugar alcohol includes at least one selected from the group consisting of mannitol, lactose, sorbitol, maltitol, xylitol, erythritol, lactitol, maltose and isomalt.

### [Ninth Aspect]

The easy-to-take granular preparation according to any one of the first to eighth aspects, wherein the granules further contains a water-insoluble polymer.

### [Tenth Aspect]

The easy-to-take granular preparation according to the ninth aspect, wherein the water-insoluble polymer is microfibrous cellulose.

### [Eleventh Aspect]

The easy-to-take granular preparation according to the tenth aspect, wherein, the microfibrous cellulose has an average fiber length from 0.01 to 2 mm and an average fiber diameter from 0.001 to 1 µm.

### [Twelfth Aspect]

The easy-to-take granular preparation according to any one of the first to eleventh aspects, wherein when the granule comes into contact with water, the granule has a hardness from 100 to 3000 N/m², and forms a gel that has adhesiveness of 500 J/m³ or less.

### [Thirteenth Aspect]

A method for producing the easy-to-take granular preparation described in any one of the first to twelfth aspects, the method including granulating a composition containing a sugar or a sugar alcohol using an aqueous solution of a thickener.

### [Fourteenth Aspect]

The method for producing the easy-to-take granular preparation according to the thirteenth aspect, the method including dry-granulating a granule obtained by the granulating and an active ingredient.

### Advantageous Effects of Invention

According to the present invention, swallowability of a granular preparation that includes granules (granulated product) containing a sugar or a sugar alcohol can be improved by coating at least a portion of the surface of the granules with a thickener. To be more specific, when such granules get in contact with moisture inside or outside of the oral cavity, the granules easily aggregate and form a gel with low adhesiveness to the inside of the oral cavity and have an appropriate level of hardness. As a result, the feeling of swallowability of the granular preparation of the present invention is improved, making the granular preparation become easy to take (easy to swallow).

In addition, the thickener is evenly distributed on the surface of the granules (granulated product) containing a sugar or sugar alcohol, or like, without being localized, and therefore, even if the amount of the thickener is increased, the granular preparation containing the granules is less likely to adhere to the inside of the oral cavity when put into the mouth.

### Brief Description of Drawings

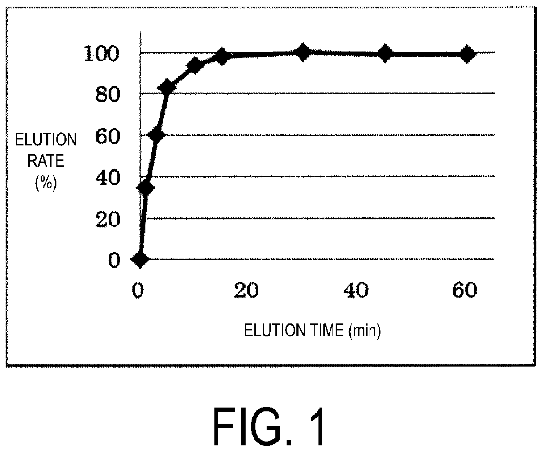

FIG. 1 indicates the results of an elution test of a granular preparation according to an embodiment of the present invention.

### Description of Embodiments

According to the Japanese Pharmacopeia 17th Edition, a granular preparation is a formulation that is granulated into a granular form and administered orally, and a powdered drug is a powdered formulation that is administered orally.

To be more specific, a granular preparation generally indicates: (i) a granular preparation obtained by adding an excipient, a binder, a disintegrating agent or other additives to an active ingredient in powder form and mixing so as to achieve a homogeneous mixture, and then forming the mixture into a granular form using an appropriate method; (ii) a granular preparation obtained by adding additives such as an excipient to a pre-granulated active ingredient, and mixing so as to obtain a homogeneous mixture, and (iii) a granular preparation obtained by adding additives such as an excipient to a pre-granulated active ingredient, mixing, and then forming the mixture into a granular form using an appropriate method. Moreover, a powdered drug indicates a drug obtained by adding an excipient or other additive to an active ingredient and mixing to obtain a homogeneous mixture.

The first aspect according to an embodiment of the present invention pertains to an easy-to-take granular preparation that includes granules containing a sugar or a sugar alcohol (may be granules made from only a sugar or a sugar alcohol) with the surface of the granules being at least partially coated with a thickener. The easy-to-take granular preparation may further contain an active ingredient or the like, but in some cases may contain only the above-mentioned granules (granulated product), which corresponds to the granules themselves according to an embodiment of the present invention. The easy-to-take granular preparation can further contain an active ingredient. Note that in the present invention, "granular preparation" also includes "powdered drugs" in addition to "granular preparations" according to the Japanese Pharmacopeia.

The easy-to-take granular preparation described above can include an active ingredient as a constituent element (constituent ingredient) separate from the granules that contain a sugar or sugar alcohol.

Alternatively, as another aspect, the active ingredient may be included in the granules coated with a thickener. In such cases, the easy-to-take granular preparation may or may not further contain an active ingredient as a constituent element that is separate from the granules.

In the granular preparation according to an embodiment of the present invention, the ease of swallowability of the granular preparation containing the granules is enhanced or improved by coating with a thickener at least a portion of, or preferably substantially the entirety of, the surface of the granules containing a sugar or sugar alcohol. When the easy-to-take granular preparation is produced by the method described in the examples herein, substantially the entire surface of the granules containing a sugar or sugar alcohol is most likely coated by the thickener.

As the sugar or sugar alcohol, any substance known in the art can be used, and examples include at least one selected from the group consisting of mannitol, lactose, sorbitol, maltitol, xylitol, erythritol, lactitol, maltose and isomalt.

In the present invention, "thickener" refers to a substance that turns into a viscous liquid upon the addition of water or the like. As described in the examples herein, at least a portion of the surface of the granules containing a sugar or sugar alcohol is coated with the thickener, and thereby when the granules come into contact with water, the granules form a gel having low adhesiveness and an appropriate level of hardness, and as a result, when the granular preparation according to an embodiment of the present invention is taken with or without water, a property of improving swallowing and creating an easy-to-drink (easy-to-swallow) state is achieved.

More specifically, as measured by the methods described herein, the granules have a hardness from 100 to 3000 N/m², and preferably from 300 to 2500 N/m², and form a gel that has adhesiveness of 500 J/m³ or less, and preferably 450 J/m³ or less.

The thickener is not particularly limited, and examples thereof include at least one water-soluble polymer selected from the group consisting of sodium carboxymethyl cellulose (also referred to as "carmellose sodium"), xanthan gum, sodium alginate, carrageenan, guar gum, and gelatin. Note that the water-soluble polymer may be a natural product or a synthetic material.

In addition to the active ingredient, the granular preparation according to an embodiment of the present invention and/or the granules contained therein may include other optional ingredients known to those skilled in the art such as, for example, the above-mentioned thickeners, binders, disintegrating agents, and excipients. For example, water-insoluble polymers can be further included to effectively improve the hardness of the granules. As the water-insoluble polymer, any water-soluble polymer known in the art can be used as long as the purpose of the present invention can be achieved, and the water-soluble polymer may be a natural product or a synthetic product.

Suitable examples of water-insoluble polymers include, for example, microfibrous cellulose, crystalline cellulose, powdered cellulose, and various cellulose derivatives, known in the art. Of these, microfibrillated cellulose or crystalline cellulose is particularly preferable.

"Microfibrous cellulose" means a cellulose that is generally produced from plant fibers and has a fiber diameter (short diameter) or thickness from several nm to around 1 µm, and for which the surface area is remarkably increased and hydrophilicity, which is an intrinsic characteristics of cellulose, is remarkably strengthened, and a three-dimensional network structure is formed through entanglement of microfibers, without compromising the basic properties (physical and chemical stability, etc.) of the raw material cellulose.

The dry substance of such microfibrous cellulose can be obtained by any conventionally known technique, including for example, directly pulverizing dry cellulose fibers using a ball mill to directly obtain the microfibrous cellulose in a dry state (JP 56-100801 A). Alternatively, a microfibrous cellulose in an aqueous suspension state constituted by microfibrous cellulose obtained by microfibrillating an aqueous dispersion of cellulose fibers using a high pressure homogenizer is subjected to solvent replacement in a replacement step, after which the solvent is removed through a drying step, and the material is then pulverized in a pulverizing step, thereby a dried product of microfibrous cellulose can be obtained (JP 2009-203559 A).

A suitable example of the microfibrous cellulose can include a microfibrous cellulose that is a fiber aggregate having an average fiber length of approximately from 0.01 to 2 mm, and an average fiber diameter of approximately from 0.001 to 1 µm, and preferably approximately from 0.01 to 0.1 µm. For example, various grades of products of such microfibrous cellulose (water-containing state with solid content from 10 to 35%) are commercially available from Daicel FineChem Ltd. under the series trade name "Celish" (average fiber diameter of approximately from 0.01 to 0.1 µm).

Moreover, representative examples of commercially available crystalline cellulose products include Avicel (FMC Corporation), Ceolus (Asahi Kasei Chemicals Corp.), and Vivapur (Rettenmaier Japan Co., Ltd.), and representative examples of powdered cellulose can include KC Flock (Nippon Paper Chemicals Co., Ltd.), ARBOCEL (Rettenmaier Japan Co., Ltd.), and Solka Floc (Kimura Sangyo Co., Ltd.).

The easy-to-take granular preparation according to an embodiment of the present invention can be used in various food product applications such as, for example, food supplements, nutraceuticals, and health foods, and in pharmaceutical applications.

Therefore, any substance known in the art can be selected, according to the various above-mentioned applications, as the active ingredient (active ingredient) included in the easy-to-take granular preparation according to an embodiment of the present invention.

For example, in the case of a food product application, various nutritional ingredients such as proteins, carbohydrates, lipids and minerals; various vitamins and their derivatives; health food materials such as microorganisms and various extracts from plants or animals; various specified additives based on Article 10 of the Food Sanitation Act or existing additives such as acidulants, sweeteners, excipients, surfactants, lubricants, adjuvants, acidulants, sweeteners, flavoring agents, spices, coloring agents, and stabilizers; and other optional ingredients acceptable as food ingredients (food additives) listed in the List of Designated Food Additives, etc. can be included.

Or, in the case of pharmaceutical applications, in addition to the active ingredient (or medicinal ingredient), as necessary, other optional pharmaceutically acceptable ingredients such as excipients, surfactants, lubricants, adjuvants, acidulants, sweeteners, flavoring agents, spices, colorants, and stabilizers can be included. As these optional ingredients, for example, the ingredients described in the Japanese Pharmaceutical Excipients Dictionary (Yakuji Nippo, Ltd.) and the Japanese Pharmacopeia can be used. Note that the applications and types of the medicinal ingredients and auxiliary agents are not particularly limited. Furthermore, as long as the desired effect of the present invention is achieved, the blending ratios of these optional ingredients is not particularly limited, and can be determined, as appropriate, by a person skilled in the art.

Examples of the above-mentioned applications and types of medicinal ingredients include drugs for the central nervous system, drugs for the peripheral nervous system, drugs for sensory organs, drugs for circulatory organs, drugs for respiratory organs, hormones, urogenital drugs, other individual organ-based pharmaceuticals, vitamins, analeptics, drugs for blood and bodily fluids, other metabolic pharmaceuticals, drugs for cellular activation, tumor drugs, radioactive pharmaceuticals, drugs for allergies, other pharmaceuticals for tissue cell function, herbal medicines, Chinese medicines, other pharmaceuticals based on herbal and Chinese medicines, antibiotic formulations, chemotherapeutic drugs, biological formulations, drugs for parasites, pharmaceuticals for other pathogenic organisms, drugs for prescription formulations, diagnostic drugs, drugs for public health, and pharmaceuticals for extracorporeal diagnostics.

In granules containing a sugar or sugar alcohol of the granular preparation according to an embodiment of the present invention, the thickness of the coating layer formed by the thickener is, for example, about 0.01 to 250 microns, and the sugar or sugar alcohol in the granules, and the thickener are typically from 50 to 99 wt.% and from 0.01 to 30 wt.%, respectively, and preferably from 60 to 99 wt.% and from 0.05 to 20 wt.%, respectively.

The size, shape, and the like of the granular preparation or granules containing the sugar or sugar alcohol according to an embodiment of the present invention are not particularly limited, but typically, the following physical properties are preferable. Note that these physical properties were measured according to the methods described below.
(1) Average particle size: from 100 to 2000 µm
(2) moisture: from 0.2 to 5.0 wt.%.

Furthermore, the content and type of the above-mentioned active ingredient and other optional ingredients included in the granular preparation or granules according to an embodiment of the present invention can be determined, as appropriate, by a person skilled in the art according to the usage purpose of the granular preparation and the characteristics of these ingredients, and the like. For example, the active ingredient can be contained at an amount from 0.01 to 80 wt.%, and preferably from 0.1 to 50 wt.%, of the total granular preparation. Also, each of the ingredients is included in any shape and form known in the art, such as granules, particles, or powders, and each is preferably included in the form of granules.

The easy-to-take granular preparation according to an embodiment of the present invention, and the granules containing a sugar or sugar alcohol and having a surface at least partially coated by a thickener can be produced by a method that includes granulating a composition containing a sugar or sugar alcohol using water or an aqueous solution of said substance through any wet granulation method known in the art. The wet granulation method is a method of forming a composite by dispersing and drying each ingredient in the presence of water, and specific examples of wet granulation include spray drying, rolling granulation, agitated granulation, and spray methods such as fluidized bed granulation, freeze drying, extrusion granulation, and kneading granulation. The easy-to-take granular preparation and granules according to an embodiment of the present invention can be produced by any of these methods known in the art. Incidentally, the concentration of the aqueous solution of the thickener can be selected, as appropriate, by a person skilled in the art, but ordinarily, an aqueous solution of 0.01 to 20 wt.% and preferably 0.1 to 15 wt.% is used.

For example, as described in the examples herein, the granules containing a sugar or sugar alcohol and having a surface that is at least partially coated by a thickener can be granulated by a method that includes spraying an aqueous solution of the thickener onto a composition containing a sugar or a sugar alcohol, etc. and granulating, or adding an aqueous solution of the thickener to a composition containing a sugar or sugar alcohol, etc. and granulating. Note that in addition to the sugar or sugar alcohol, if an active ingredient and other ingredients are to also be included in the granules, a composition containing the additional ingredients described above is prepared, as appropriate, by adding the additional ingredients at any stage, such as before or during the granulation described above.

Furthermore, the granular preparation according to an embodiment of the present invention can be produced by treating, as appropriate and as necessary, the granules (granulated product) obtained as described above and the other ingredients such as the active ingredient by an appropriate means and method known in the art, such as mixing or granulation. For example, the production method according to an embodiment of the present invention can include dry-granulating the granules obtained through granulation and the active ingredient, etc. using any method or means known in the art (for example, the granules and active ingredient are mixed and compression molded into a sheet form, and then pulverized to form a granular preparation). Alternatively, the active ingredient (the active ingredient may be in the form of granules, or powder, etc.) may be added to the granules and mixed therewith without granulating using the granules and the active ingredient.

Note that all of the details described in the prior art documents cited in the present specification are incorporated herein as references.

### Examples

Hereinafter, the present invention is more specifically described through examples, but the present invention is not limited by these examples.

### [Evaluation of Average Particle Size, Moisture, Hardness, and Adhesiveness]

For each granular preparation (granule) obtained in the following examples and comparative examples, physical property values were measured using the following conditions and methods.

Average particle size: Measured using a laser diffraction device (LA-960, available from Horiba, Ltd.). The measurement was taken three times each, and the average value of the three measurements was used as the measurement result.

Moisture: 5 grams of granules containing sugar or sugar alcohol were heated to 105°C until a moisture change rate reached 0.05%/min, and the moisture was measured using a halogen moisture measuring instrument (model HB43 available from Mettler-Toledo Co., Ltd.).

Hardness and adhesiveness: Measured using a texture analyzer (TA.XTPlus, available from Stable Micro Systems). In addition, as the jig, a 20-8 probe (20 mmcp probe, height of 8 mm, made of resin) and an F-245 (measuring container, diameter of 40 mm, height of 15 mm), which are jigs for measuring food products for persons with dysphagia in compliance with the standard of the Consumer Affairs Agency of Japan, were used.

An amount of 6.5 g of a measurement substance (granules or granular preparation) and 6.5 g of purified water were added to the F-245 jig, and then lightly mixed so that the mixture is well blended. Next, a compression test was performed twice at a travel distance setting of 15 mm from a position of a 20 mm clearance at a test speed of 1.0 mm/sec. The hardness (N/m²) was calculated from an obtained maximum stress value, and the adhesiveness (J/m³) was calculated from the stress value when the probe returned. Measurements were taken three times each, and the average value of the measurements was used as the measurement result.

### [Sensory Evaluation]

Furthermore, a sensory evaluation was implemented as follows in the following examples.

Cohesiveness in texture: Four adult men and women took the granular preparation without water, and the cohesive texture was evaluated according to the following five-level criteria. The average values are listed in the table.
1: Not detected; very weak
2: Slightly detected; weak
3: Clearly detected
4: Somewhat strongly detected
5: Very strongly detected

Post-gelled hardness: Four adult men and women took the granular preparation without water, and evaluated the hardness after turning into gels according to the following five-level criteria. The average values are listed in the table.
1: Very soft; no different than water
2: Soft
3: Appropriate hardness
4: Somewhat hard
5: Very hard

Adhesiveness: Four adult men and women took the granular preparation without water, and evaluated the adhesiveness according to the following five-level criteria. The average values are listed in the table.
1: Not detected; very weak
2: Slightly detected; weak
3: Clearly detected
4: Somewhat strongly detected
5: Very strongly detected

### [Elution Time]

Using water as the eluate, the elution time was measured in accordance with the "Elution Test Method" (rotational basket method) described in Japanese Pharmacopeia. The elution rate of acetaminophen into the eluate was measured by comparing the absorbance at a wavelength of 243 nm of a test solution, measured by a UV-visible light absorbance measurement method, with absorbance at a wavelength of 243 nm of a standard solution prepared by dissolving acetaminophen (acetaminophen, available from Iwaki Seiyaku Co., Ltd.) in water such that the concentration became approximately the same as the concentration of acetaminophen included in the eluate.

### Comparative Example 1

A granulated product was obtained by adding 145.5 g of mannitol (PEARLITOL, Roquette) and 4.5 g of carmellose sodium (CMC Daicel, Daicel FineChem Ltd.) to a fluidized bed granulator (FL-LABO, Freund Corporation), spraying purified water at a rate of 3.8 g/min, and granulating. The obtained granulated product had the following physical property values.
Moisture value: 0.65%, average particle size: 285 µm

### Comparative Example 2

90 g of mannitol (PEARLITOL, Roquette), 2 g of hydroxypropyl cellulose (HPC-SSL, Nippon Soda Co., Ltd.) and 5.5 g of hydroxypropyl starch (HPS-101W, Freund Corporation) were mixed in a mortar, 15 mL of purified water was added thereto, and the mixture was kneaded. The kneaded product was pressed against a sieve having openings of 500 µm to form granule shapes, and then dried using a dryer to obtain a granulated product. The obtained granulated product had the following physical property values.
Moisture value: 0.76%, average particle size: 848 µm

### Comparative Example 3

187.0 g of mannitol (PEARLITOL, Roquette), 1.0 g of hydroxypropyl methylcellulose (ISP), and 11.0 g of hydroxypropyl starch (HPS-101W, Freund Corporation) were mixed, 30 mL of purified water was added thereto, and the mixture was kneaded. The kneaded product was granulated using an extrusion granulator (Multi Granulator Model MG-55-2, Dalton Corporation), and then dried with a dryer to obtain a granulated product. The obtained granulated product had the following physical property values.
Moisture value: 0.64%, average particle size: 770 µm

### Comparative Example 4

187.0 g of lactose (GranuLac 200, Meggle AG), 1.0 g of hydroxypropyl methylcellulose (ISP), and 11.0 g of hydroxypropyl starch (HPS-101W, Freund Corporation) were mixed, 25 mL of purified water was added thereto, and the mixture was kneaded. The kneaded product was granulated using an extrusion granulator (Multi Granulator Model MG-55-2, Dalton Corporation), and then dried with a dryer to obtain a granulated product. The obtained granulated product had the following physical property values.
Moisture value: 1.25%, average particle size: 973 µm

### Comparative Example 5

184.0 g of lactose (GranuLac 200, Meggle AG), 4.0 g of hydroxypropyl cellulose (HPC-SSL, Nippon Soda Co., Ltd.) and 11.0 g of hydroxypropyl starch (HPS-101W, Freund Corporation) were mixed, 20 mL of purified water was added thereto, and the mixture was kneaded. The kneaded product was granulated using an extrusion granulator (Multi Granulator Model MG-55-2, Dalton Corporation), and then dried with a dryer to obtain a granulated product. The obtained granulated product had the following physical property values.
Moisture value: 0.50%, average particle size: 1009 µm

### Example 1

145.5 g of mannitol (PEARLITOL, Roquette) was added to a fluidized bed granulator (FL-LABO, Freund Corporation), and granulation was carried out by spraying, at a rate of 3.0 g/min, 450 g of a CMCNa aqueous solution obtained by dissolving carmellose sodium (CMC Daicel, Daicel FineChem Ltd.) in water to 1.0 wt.%, and a granulated product was thereby obtained. The obtained granulated product had the following physical property values.
Moisture value: 0.39%, average particle size: 383 µm

### Example 2

108.0 g of mannitol (PEARLITOL, Roquette) and 37.5 g of ascorbic acid (vitamin C, Iwaki Seiyaku Co., Ltd.) were added to a fluidized bed granulator (FL-LABO, Freund Corporation), and granulation was carried out by spraying, at a rate of 3.0 g/min, 450 g of a CMCNa aqueous solution obtained by dissolving 4.5 g of carmellose sodium (CMC Daicel, Daicel FineChem Ltd.) in water to 1.0 wt.%, and a granulated product was thereby obtained. The obtained granulated product had the following physical property values.
Moisture value: 0.44%, average particle size: 409 µm

### Example 3

20.0 g of ascorbic acid (vitamin C, Iwaki Seiyaku Co., Ltd.) was added to 80.0 g of the granules produced in Example 1, after which the mixture was mixed well, and a granular preparation was obtained. The obtained granular preparation had the following physical properties values.
Moisture value: 0.51%, average particle size: 447 µm

### Example 4

291.0 g of mannitol (PEARLITOL, Roquette) and 8.0 g of carmellose sodium (CMC Daicel, Daicel FineChem Ltd.) were mixed, and 50 g of a CMCNa aqueous solution obtained by dissolving 1.0 g of carmellose sodium (CMC Daicel, Daicel FineChem Ltd.) in water to 2.0 wt.% was added thereto, and the mixture was kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 1.33%, average particle size: 1213 µm

### Example 5

291.0 g of lactose (GranuLac 200, Meggle AG) and 8.2 g of carmellose sodium (CMC Daicel, Daicel FineChem Ltd.) were mixed, and 40 g of a CMCNa aqueous solution obtained by dissolving 0.8 g of carmellose sodium (CMC Daicel, Daicel FineChem Ltd.) in water to 2.0 wt.% was added thereto, and the mixture was kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 1.24%, average particle size: 1219 µm

### Example 6

291.0 g of erythritol (Erythritol 50M, B Food Science Co., Ltd.) and 8.4 g of carmellose sodium (CMC Daicel, Daicel FineChem Ltd.) were mixed, and 30 g of a CMCNa aqueous solution obtained by dissolving 0.6 g of carmellose sodium (CMC Daicel, Daicel FineChem Ltd.) in water to 2.0 wt.% was added thereto, and the mixture was kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 0.45%, average particle size: 1199 µm

### Example 7

291.0 g of xylitol (Xylit P, B Food Science Co., Ltd.) and 8.6 g of carmellose sodium (CMC Daicel, Daicel FineChem Ltd.) were mixed, and 20 g of a CMCNa aqueous solution obtained by dissolving 0.4 g of carmellose sodium (CMC Daicel, Daicel FineChem Ltd.) in water to 2.0 wt.% was added thereto, and the mixture was kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 0.31%, average particle size: 1282 µm

### Example 8

194.0 g of maltose (Nisshoku crystalline maltose, Nihon Shokuhin Kako Co., Ltd.) and 5.68 g of carmellose sodium (CMC Daicel, Daicel FineChem Ltd.) were mixed, and 16 g of a CMCNa aqueous solution obtained by dissolving 0.32 g of carmellose sodium (CMC Daicel, Daicel FineChem Ltd.) in water to 2.0 wt.%, and 2 g of purified water were added thereto, and the mixture was kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 0.52%, average particle size: 1387 µm

### Example 9

5.0 g of caffeine (caffeine extract, Shiratori Pharmaceutical Co., Ltd.) was added to 95.0 g of the granules produced in Example 5, and then mixed well, and a granular preparation was obtained. The obtained granular preparation had the following physical properties values.
Moisture value: 1.21%, average particle size: 1153 µm

### Example 10

5.0 g of caffeine (caffeine extract, Shiratori Pharmaceutical Co., Ltd.) was added to 95.0 g of the granules produced in Example 6, and then mixed well, and a granular preparation was obtained. The obtained granular preparation had the following physical properties values.
Moisture value: 0.34%, average particle size: 1195 µm

### Example 11

5.0 g of caffeine (caffeine extract, Shiratori Pharmaceutical Co., Ltd.) was added to 95.0 g of the granules produced in Example 7, and then mixed well, and a granular preparation was obtained. The obtained granular preparation had the following physical properties values.
Moisture value: 0.34%, average particle size: 1273 µm

### Example 12

5.0 g of caffeine (caffeine extract, Shiratori Pharmaceutical Co., Ltd.) was added to 95.0 g of the granules produced in Example 8, and then mixed well, and a granular preparation was obtained. The obtained granular preparation had the following physical properties values.
Moisture value: 0.45%, average particle size: 1281 µm

### Example 13

282.0 g of mannitol (PEARLITOL, Roquette), 8.0 g of carmellose sodium (CMC Daicel, Daicel FineChem Ltd.), and 9.0 g of crystalline cellulose (Ceolus PH-101, Asahi Kasei Chemicals Corp.) were mixed, and 50 g of a CMCNa aqueous solution obtained by dissolving 1.0 g of carmellose sodium (CMC Daicel, Daicel FineChem Ltd.) in water to 2.0 wt.% was added thereto, and the mixture was kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 1.24%, average particle size: 1269 µm

### Example 14

261.0 g of mannitol (PEARLITOL, Roquette), 8.0 g of carmellose sodium (CMC Daicel, Daicel FineChem Ltd.), and 30.0 g of crystalline cellulose (Ceolus PH-101, Asahi Kasei Chemicals Corp.) were mixed, and 50 g of a CMCNa aqueous solution obtained by dissolving 1.0 g of carmellose sodium (CMC Daicel, Daicel FineChem Ltd.) in water to 2.0 wt.%, and 5 g of purified water were added thereto, and the mixture was kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 1.62%, average particle size: 1234 µm

### Example 15

278.5 g of mannitol (PEARLITOL, Roquette), 9.0 g of carmellose sodium (CMC Daicel, Daicel FineChem Ltd.), and 62.5 g of a damp body of microfibrous cellulose (Celish, Daicel FineChem Ltd.) were mixed and kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 1.67%, average particle size: 1315 µm

### Example 16

60.0 g of ascorbic acid (vitamin C, Iwaki Seiyaku Co., Ltd.) was added to 240.0 g of the granules produced in Example 1 and mixed well. Next, the mixture was molded with a dry granulator (TF-LABO, Freund Corporation), the grain size was made uniform using an oscillator type grain sizer, and a granulated product was thereby obtained. The obtained granulated product (granular preparation) had the following physical property values.
Moisture value: 0.71%, average particle size: 525 µm

### Example 17

60.0 g of N-acetylglucosamine (Marine Sweet YSK, Yaizu Suisankagaku Industry Co., Ltd.) was added to 240.0 g of the granules produced in Example 1 and mixed together. Next, the mixture was molded with a dry granulator (TF-LABO, Freund Corporation), the grain size was made uniform using an oscillator type grain sizer, and a granulated product was thereby obtained. The obtained granulated product (granular preparation) had the following physical property values.
Moisture value: 0.60%, average particle size: 579 µm

### Example 18

582.0 g of mannitol (PEARLITOL, Roquette) and 16.95 g of carmellose sodium (CMC Daicel, Daicel FineChem Ltd.) were mixed, and while adding 90 g of a CMCNa aqueous solution obtained by dissolving 1.35 g of carmellose sodium (CMC Daicel, Daicel FineChem Ltd.) in water to 1.5 wt.%, the mixture was granulated using an agitation granulator (High-speed mixer FS-GS-5, Fukae Powtec Co., Ltd.). Next, the material was dried with a dryer, the grain size was made uniform, and a granulated product was obtained. The obtained granulated product had the following physical property values.
Moisture value: 0.44%, average particle size: 188 µm

### Example 19

558.0 g of mannitol (PEARLITOL, Roquette) and 40.65 g of carmellose sodium (CMC Daicel, Daicel FineChem Ltd.) were mixed, and while adding 90 g of a CMCNa aqueous solution obtained by dissolving 1.35 g of carmellose sodium (CMC Daicel, Daicel FineChem Ltd.) in water to 1.5 wt.%, the mixture was granulated using an agitation granulator (High-speed mixer FS-GS-5, Fukae Powtec Co., Ltd.). Next, material was dried with a dryer, the grain size was made uniform, and a granulated product was obtained. 80.0 g of N-acetylglucosamine (Marine Sweet YSK, Yaizu Suisankagaku Industry Co., Ltd.) was added to 20.0 g of the obtained granulated product, and then mixed well, and a granular preparation was obtained. The obtained granular preparation had the following physical properties values.
Moisture value: 0.75%, average particle size: 151 µm

### Example 20

582.0 g of erythritol (erythritol 50M, B Food Science Co., Ltd.) and 17.4 g of carmellose sodium (CMC Daicel, Daicel FineChem Ltd.) were mixed, and while adding 40 g of a CMCNa aqueous solution obtained by dissolving 0.6 g of carmellose sodium (CMC Daicel, Daicel FineChem Ltd.) in water to 1.5 wt.%, the mixture was granulated using an agitation granulator (High-speed mixer FS-GS-5, Fukae Powtec Co., Ltd.). Next, the material was dried with a dryer, the grain size was made uniform, and a granulated product was obtained. The obtained granulated product had the following physical property values.
Moisture value: 0.54%, average particle size: 156 µm

### Example 21

582.0 g of mannitol (PEARLITOL, Roquette) and 16.65 g of xanthan gum (Echo Gum, DSP Gokyo Food & Chemical Co., Ltd.) were mixed and while adding 90 g of a xanthan gum aqueous solution obtained by dissolving 1.35 g of xanthan gum (Echo Gum, DSP Gokyo Food & Chemical Co., Ltd.) in water to 1.5 wt.%, the mixture was granulated using an agitation granulator (High-speed mixer FS-GS-5, Fukae Powtec Co., Ltd.). Next, the material was dried with a dryer, the grain size was made uniform, and a granulated product was obtained. The obtained granulated product had the following physical property values.
Moisture value: 2.92%, average particle size: 431 µm

### Example 22

5.0 g of caffeine (caffeine extract, Shiratori Pharmaceutical Co., Ltd.) was added to 95.0 g of the granules produced in Example 21, and then mixed well, and a granular preparation was obtained. The obtained granular preparation had the following physical properties values.
Moisture value: 2.61%, average particle size: 418 µm

### [Extrusion Granulation Without Using an Aqueous Solution of a Thickener]

In the production of granules by extrusion granulation, if the thickener that is used is one or more water-soluble polymers selected from the group consisting of sodium carboxymethyl cellulose, xanthan gum, sodium alginate, carrageenan, guar gum, and gelatin, even granules produced by adding purified water without using an aqueous solution of the thickener form a gel that has low adhesiveness and appropriate hardness when contacted with water.

### Example 23

291.0 g of mannitol (PEARLITOL, Roquette) and 9.0 g of carmellose sodium (CMC Daicel, Daicel FineChem Ltd.) were mixed, 50 g of purified water was added thereto, and the mixture was kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 0.43%, average particle size: 769 µm

### Example 24

291.0 g of lactose (GranuLac 200, Meggle AG) and 9.0 g of carmellose sodium (CMC Daicel, Daicel FineChem Ltd.) were mixed, 40 g of purified water was added thereto, and the mixture was kneaded. The kneaded product was granulated using an extrusion granulator (Multi Granulator Model MG-55-2, Dalton Corporation), and then dried with a dryer. Next, the grain size was made uniform, and a granulated product was thereby obtained. The obtained granulated product had the following physical property values.
Moisture value: 0.94%, average particle size: 783 µm

### Example 25

5.0 g of caffeine (caffeine extract, Shiratori Pharmaceutical Co., Ltd.) was added to 95.0 g of the granules produced in Example 23, and then mixed well, and a granular preparation was obtained. The obtained granular preparation had the following physical properties values.
Moisture value: 0.40%, average particle size: 762 µm

### [Measurement of hardness and adhesiveness]

The hardness and adhesiveness of the granulated products (granules) obtained in Examples 1 and 2 and Comparative Examples 1 and 2 were measured, and the measurement results are shown in Table 1 along with the sensory evaluation results. The hardness and adhesiveness of the granulated products (granules and granular preparations) obtained in Example 3 to 25 and Comparative Examples 3 to 5 were measured, and the measurement results are shown in Table 1.

**[Table 1]**

| | Evaluation with Texture Analyzer | | Sensory Evaluation (Average Value) | | |
|---|---|---|---|---|---|
| | Hardness (N/m²) | Adhesiveness (J/m³) | Cohesiveness in texture | Hardness after gelled | Adhesiveness in oral cavity |
| Example 1 | 645 | 167 | 3.5 | 3.0 | 2.8 |
| Example 2 | 623 | 186 | 3.3 | 2.4 | 2.3 |
| Example 3 | 531 | 192 | - | - | - |
| Example 4 | 1333 | 74 | - | - | - |
| Example 5 | 1147 | 93 | - | - | - |
| Example 6 | 471 | 65 | - | - | - |
| Example 7 | 266 | 31 | - | - | - |
| Example 8 | 1274 | 40 | - | - | - |
| Example 9 | 1030 | 84 | - | - | - |
| Example 10 | 405 | 55 | - | - | - |
| Example 11 | 274 | 32 | - | - | - |
| Example 12 | 1159 | 41 | - | - | - |
| Example 13 | 1680 | 126 | - | - | - |
| Example 14 | 2849 | 249 | - | - | - |
| Example 15 | 1638 | 93 | - | - | - |
| Example 16 | 389 | 52 | - | - | - |
| Example 17 | 388 | 72 | - | - | - |
| Example 18 | 808 | 95 | - | - | - |
| Example 19 | 729 | 101 | - | - | - |
| Example 20 | 760 | 67 | - | - | - |
| Example 21 | 1111 | 239 | - | - | - |
| Example 22 | 926 | 196 | - | - | - |
| Example 23 | 1133 | 85 | - | - | - |
| Example 24 | 1152 | 81 | - | - | - |
| Example 25 | 871 | 60 | - | - | - |
| Comparative Example 1 | 3662 | 641 | 4.0 | 4.4 | 4.0 |
| Comparative Example 2 | 50 | 12 | 1.8 | 1.0 | 1.8 |
| Comparative Example 3 | 56 | 12 | - | - | - |
| Comparative Example 4 | 61 | 13 | - | - | - |
| Comparative Example 5 | 59 | 12 | - | - | - |

From the results shown in Table 1, it was demonstrated that unlike the case in which the thickener was as a powder without using an aqueous solution of the thickener (Comparative Example 1), and the case in which, like the invention described in Patent Document 3, a sugar alcohol and a thickener (hydroxypropyl cellulose and hydroxypropyl starch) were simply mixed in a mortar without using an aqueous solution of the thickener, after which water was added, and the mixture was kneaded and granulated (Comparative Example 2), when brought into contact with water, the granules and granular preparation (granulated product) according to embodiments of the present invention became a gel form having an appropriate level of hardness (from 100 to 3000 N/m², preferably from 300 to 2500 N/m²), and low adhesiveness (adhesiveness of 500 J/m³ or less, preferably 450 J/m³ or less), and an easy-to-drink granular preparation was formed.

### Example 26

8 g of the granules (granulated product) obtained in Example 1, and 2 g of acetaminophen (powder form) were mixed to obtain a granular preparation. The results of an elution test of the obtained granular preparation are shown in FIG. 1. The results demonstrated that the granular preparation according to an embodiment of the present invention had a 30 minute elution rate of 200 mg/g acetaminophen of 80% or higher, which complies with the standard.

### Industrial Applicability

The present invention contributes greatly to the research and development of easy-to-take granular preparations.

## Claims

1. An easy-to-take granular preparation comprising a granule that contains a sugar or a sugar alcohol, the granule having a surface at least partially coated with a thickener.

2. The easy-to-take granular preparation according to claim 1, further comprising an active ingredient.

3. The easy-to-take granular preparation according to claim 2, comprising an active ingredient as a constituent element separate from the granule.

4. The easy-to-take granular preparation according to claim 2 or 3, further comprising an active ingredient in a granule coated with a thickener.

5. The easy-to-take granular preparation according to any one of claims 1 to 4, wherein substantially the entire surface of the granule is coated with a thickener.

6. The easy-to-take granular preparation according to any one of claims 1 to 5, wherein the thickener comprises at least one water-soluble polymer selected from the group consisting of sodium carboxymethyl cellulose, xanthan gum, sodium alginate, carrageenan, guar gum, and gelatin.

7. The easy-to-take granular preparation according to claim 6, wherein the water-soluble polymer is sodium carboxymethyl cellulose.

8. The easy-to-take granular preparation according to any one of claims 1 to 7, wherein the sugar or sugar alcohol includes at least one selected from the group consisting of mannitol, lactose, sorbitol, maltitol, xylitol, erythritol, lactitol, maltose and isomalt.

9. The easy-to-take granular preparation according to any one of claims 1 to 8, wherein the granule further contains a water-insoluble polymer.

10. The easy-to-take granular preparation according to claim 9, wherein the water-insoluble polymer is microfibrous cellulose.

11. The easy-to-take granular preparation according to claim 10, wherein the microfibrous cellulose has an average fiber length from 0.01 to 2 mm and an average fiber diameter from 0.001 to 1 µm.

12. The easy-to-take granular preparation according to any one of claims 1 to 11, wherein when the granule comes into contact with water, the granule has a hardness from 100 to 3000 N/m² and forms a gel that has adhesiveness of 500 J/m³ or less.

13. A method for producing the easy-to-take granular preparation described in any one of claims 1 to 12, the method comprising granulating a composition containing a sugar or a sugar alcohol using an aqueous solution of a thickener.

14. The method for producing the easy-to-take granular preparation according to claim 13, the method comprising dry-granulating a granule obtained by the granulating and an active ingredient.
